(19) Europäisches Patentamt
European Patent Office
Office européen des brevets

(11) EP 0 810 829 B1

(12) EUROPEAN PATENT SPECIFICATION

(45) Date of publication and mention
of the grant of the patent:
**12.04.2000 Bulletin 2000/15**

(51) Int Cl.[7]: **A23L 1/305**, A61K 31/195

(86) International application number:
**PCT/EP96/00739**

(21) Application number: **96904849.5**

(22) Date of filing: **22.02.1996**

(87) International publication number:
**WO 96/25861 (29.08.1996 Gazette 1996/39)**

(54) **AMINO ACID COMPOSITIONS AND USE THEREOF IN CLINICAL NUTRITION**

AMINOSÄURENZUSAMMENSETZUNGEN UND VERWENDUNG DERSELBEN KLINISCHEN
NÄHRUNG

COMPOSITIONS D'ACIDES AMINES ET LEUR UTILISATION DANS LA NUTRITION CLINIQUE

(84) Designated Contracting States:
**AT BE CH DE DK ES FR GB GR IE IT LI LU NL PT
SE**
Designated Extension States:
**SI**

(30) Priority: **23.02.1995 US 392694**
**14.06.1995 GB 9512100**

(43) Date of publication of application:
**10.12.1997 Bulletin 1997/50**

(73) Proprietor: **Novartis Nutrition AG**
**3001 Bern (CH)**

(72) Inventor: **SCHNEIDER, Heinz**
**CH-1792 Cordast (CH)**

(56) References cited:
**EP-A- 0 614 616          WO-A-93/05780**
**FR-A- 2 591 893          US-A- 4 988 724**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The present invention relates to the use of specific amino acids in the preparation of a medicament or nutritional formulation which may be therapeutically administered to patients suffering from a wide variety of diseases and morbid conditions.

WO-A-9305780 discloses total parenteral nutrition formulations (TPN) essentially free of arginine for preventing or inhibiting systemic hypotension induced by a biological response modifier or by bacterial sepsis. The TPN formulations of consist basically of a mixture of 15 or more amino acids, glucose and dextrose and optionally trace elements, vitamin supplements and essential salts.

EP-A-0614616 discloses enteral formulations which are specifically designed for treating patients with liver diseases. The enteral formulations contain i.a. a mixture of 18 amino acids in free crystalline amino acid form.

US-A-4988724 discloses total parenteral nutrition formulations which include 8 essential amino acids together with at least one non-essential amino acid and a very small amount of arginine for use in the detection of recurrent malignant disease in patients.

It has now surprisingly been found that, *inter alia,* glycine is suitable for minimizing and/or preventing the metabolic effects of a wide range of disease states and traumatized or other morbid conditions induced by elevated TNF levels.

In view of the above-mentioned effects, there are provided pharmaceutical compositions, formulations and diets comprising glycine as well as methods of using glycine. For use in the compositions, formulations, diets and methods of the invention, glycine is conveniently employed in free amino acid form, in the form of glycine precursors, in particular alanine or serine, likewise in free amino acid form, in physiologically acceptable salt form of said amino acids, or in form of mixtures of said amino acids and/or physiologically acceptable salts thereof. Glycine is preferably used in free amino acid form, in physiologically acceptable salt form or in the form of a mixture of glycine in free amino acid form with glycine in physiologically acceptable salt form; most preferably glycine is in free amino acid form.

The term "amino acid of the invention" as used hereinafter is meant to refer to glycine, alanine and/or serine, in free amino acid form and/or physiologically acceptable salt form.

The invention therefore provides the use of at least one amino acid selected from the group consisting of glycine, alanine and serine, or the physiologically acceptable salts thereof, in the preparation of a medicament or nutritional formulation for enteral administration for the diminution of tumor necrosis factor (TNF) levels in patients in whom said levels are elevated beyond those which mediate physiological homeostasis and local inflammation. Such diminuition of TNF levels can i.a. be achieved by inhibition or diminuition of:

(i) Tumour necrosis factor (TNF) production by macrophage-type cells;
(ii) the release of TNF from macrophage-type cells; and/or
(iii) the binding of TNF by TNF receptors.

By "elevated beyond those which mediate physiological homeostasis and local inflammation" is meant an amount of TNF which is more than the minimum required to regulate circadian rhythm of body temperature, sleep and appetite or the minimum required to exert autocrine and paracrine effects in cells neighboring those in which the TNF is produced or secreted, absent systemic inflammatory effects remote from such cells.

The invention still further provides the use of at least one amino acid, selected from the group consisting of glycine, alanine and serine, or the physiologically acceptable salts thereof, in the preparation of a medicament or nutritional formulation to prevent or diminish the macrophage-type cell induced - TNF mediated - production of at least one protein selected from interleukin 1, interleukin 2, interleukin 6, interleukin 8, interleukin 10, endothelial, platelet and leukocyte selectin, leukocyte function associated antigen 1, very late activation antigen 4, intercellular adhesion molecules, platelet factor 4, neutrophil attractant/activation protein, Sialyl-Lewis-X, gamma interferon-induced peptides, macrophage inflammatory proteins alpha and beta, epithelium derived neutrophil attractant, granulocyte chemotactic protein 2, monocyte chemotactic protein 1, vascular cell adhesion molecule 1, and lymphocyte functional antigen 3.

Whilst the use according to the invention relates particularly to those macrophage-type cells located in the blood (monocytes or mono-nuclear phagocytes), liver (Kupffer cells), nervous system (microglial cells), digestive system (mesentery/gut), heart, kidney and bone (bone cell-derived macrophages), all cells which produce, bind or release TNF are targets for the amino acids of the invention. In particular it is envisaged that production by, release from or binding of TNF to lung derived or located macrophages will be affected by administration to the patient of the medicament or formulation according to the invention.

The invention also provides a method of reducing the risk of death following endotoxic shock and/or hypoxia-reperfusion injury, in particular, in trauma (polytrauma, burn and post-operative patients as well as septic patients) comprising administering effective amounts of an amino acid of the invention. Endotoxic shock and hypoxia-reperfusion injury are both conditions which may result in death of the patient and which involve elevated TNF levels.

The invention furthermore provides the use of at least one amino acid, selected from the group consisting of glycine, alanine and serine, or the physiologically acceptable salts thereof, in the preparation of a medicament or nutritional formulation for preventing or reducing the risk of liver disease due to excessive alcohol consumption and intestinal and pancreatic disorders resulting therefrom in patients in need of such treatment. For this indication the use of glycine and/or serine or physiologically acceptable salts thereof is preferred and the use of glycine or physiologically acceptable salts thereof is particularly preferred.

Still further the invention provides use of at least one amino acid, selected from the group consisting of glycine, alanine and serine, or the physiologically acceptable salts thereof, in the preparation of a medicament or nutritional formulation for preventing or reducing ethanol toxicity in patients in need of such treatment. For this indication the use of glycine and/or serine or physiologically acceptable salts thereof is preferred and the use of glycine or physiologically acceptable salts thereof is particularly preferred.

The invention also provides use of at least one amino acid, selected from the group consisting of glycine, alanine and serine, or the physiologically acceptable salts thereof, in the preparation of a medicament or nutritional formulation for eliminating ethanol from the stomach before entering the systemic blood circulation in patients in need of such treatment.

The invention also provides the use of an amino acid of the invention as additive to foods, soft drinks, vitamins or pharmaceutical preparations for treatment of ethanol toxicity and a method of preventing ethanol toxicity employing an effective amount of an amino acid of the invention.

It is preferred that the medicament or formulation is administered to the patient in such an amount that the amino acid of the invention diminishes TNF levels in the circulatory system to below 250pg/ml (± 10%), particularly in the case of traumatized patients suffering from severe (second or third degree) burns, in whom there may have been some leakage of bacterial endotoxins into the general circulation absent the overt clinical manifestations of sepsis. It is more preferred that the amino acid of the invention diminishes TNF levels in the circulatory system to below 80pg/ml (± 10%), and still more preferred that the TNF levels are diminished to below 20pg/ml (± 10%). The skilled man is well aware of how such levels may be detected, in particular *via* known enzyme linked immunosorbant or radioimmuno assays.

The present invention also provides the use of at least one amino acid, selected from the group consisting of glycine, alanine and serine, or the physiologically acceptable salts thereof, in the preparation of a medicament or nutritional formulation for the prevention or diminution of TNF-induced (acturial) allograft rejection. The allograft may be associated with any organ, but the most profound benefits to the patient will be observed in the case of transplantation or injection surgery associated with the lung, heart, liver, gut/mesentery and kidney.

In the case that the medicament or formulation is to be administered to transplant patients, it may advantageously further comprise at least one of the following compounds: neutralizing antibodies raised against TNF, TNF soluble receptors or derivatives or parts thereof, and cyclic polypeptide immuno-suppressants. An example of a well known cyclic polypeptide immuno-suppressant suitable for inclusion in the medicament is cyclosporin, or an effective derivative thereof. A particularly suitable TNF soluble receptor comprises a synthetic dimeric soluble fusion protein construct which combines two p60 TNF receptors with the Fc portion of an IgG molecule. This fusion protein construct possesses a higher affinity and is a superior inhibitor of TNF bioactivity than either native monomeric TNF receptors (p80 or p60) or anti-TNF antibodies.

The nutritional formulation or medicament may be administered either prophylactically, e.g. preoperatively, in the acute phase, e.g. postoperatively, or both.

The nutritional formulation or medicament may be administered to the patient enterally or parenterally. The enteral administration route is preferred, particularly for subsequent or prophylactic treatment; particularly contemplated enteral administration routes are oral administration, nasal administration and/or tube feeding. The medicament or formulation is conveniently administered in the form of an aqueous liquid. The medicament or formulation in a form suitable for enteral application is accordingly preferably aqueous or in powder form, whereby the powder is conveniently added to water prior to use. For use in tube feeding, the amount of water to be added will depend, *inter alia,* on the patient's fluid requirements and condition. It will be appreciated that, for acute treatment, the parenteral application route is preferred. The parenteral application route is, for example, also indicated where the objective is to control the effects of chronic endotoxemia.

The medicament or formulation may be so formulated as to deliver to the patient from 1 to 80g of the amino acid of the invention per 24 hours. The amount of medicament or formulation to be administered depends to a large extent on the patients's specific requirements. Such daily amounts of amino acid of the invention are suitable for treatment of the desired effects as well as for prophylactic/pretreatment. In the case that the medicament or formulation comprises a single amino acid of the invention (in the L-configuration), it may be administered to the patient in an amount such that the concentration of that amino acid in the patients's plasma is elevated to between 0.5 and 2.0 mM, preferably from 1.0 to 2.0 mM. Whilst concentrations higher than this are anticipated, it is expected that significant clinical effects will be obtained if the concentration of the acid is increased, as a consequence of administration of the formulation or medicament, so that it lies in the range of from 1.2 to 1.5mM. In traumatic, hypercatabolic patients it may even be beneficial to raise the plasma glycine, serine or alanine levels to about 0.2 to 0.3 mM which corresponds to plasma glycine levels of healthy individuals.

The most preferred amino acid of the invention for incorporation into the medicament or formulation for use according to the invention is glycine or a physiologically acceptable salt thereof.

Generally, it is indicated to use an amino acid of the invention in combination with one or more of the following components:

(i) omega-3 polyunsaturated fatty acids (PUFAs) where desired in admixture with omega-6 PUFAs;

(ii) L-arginine or other physiologically acceptable compounds associated with the synthesis of polyamines, or mixtures thereof; and

(iii) a nucleobase source.

Whereby the use of a medicament or nutritional formulation comprising an amino acid of the invention in combination with arginine or other physiologically acceptable compounds associated with the synthesis of polyamines such as ornithine is preferred. Use of a medicament or nutritional formulation comprising an amino acid of the invention, arginine or ornithine and omega-3 polyunsaturated fatty acids (PUFAs) is also preferred.

Nucleobase sources suitable for use in combination with the amino acids of the invention comprise or consist of natural nucleobases, nucleosides, nucleotides, RNA, DNA, equivalents thereof and/of mixtures comprising one or more of these compounds.

Natural nucleobases include the purines adenine and guanine as well as the pyrimidines cytosine, thymine and uracil. Where the nucleobase source is in the form of free nucleobases, it is preferably uracil.

Natural nucleosides include the ribose nucleosides adenosine, guanosine, uridine and cytidine and the deoxyribose nucleosides deoxyadenosine, deoxyguanosine, deoxythymidine and deoxycytidine.

Natural nucleotides include phosphate esters of natural nucleosides, such as the monophosphates adenylate (AMP), guanylate (GMP), uridylate (UMP), cytidylate (CMP), deoxythymidiylate (dTMP), deoxycytidylate (dCMP), and diphosphates and triphosphates of natural nucleosides such as ADP and ATP.

A purified nucleobase source, such as yeast is preferred. However, other sources such as meat and the like may be used. Preferably the nucleobase source is RNA.

Accordingly, the invention provides medicaments or nutritional formulations comprising effective amounts of:

(a) an amino acid of the invention (component (a)) in association with one or more components selected from
(b) omega-3 PUFAs where desired in admixture with omega-6 PUFAs (component (b));
(c) L-arginine or other physiologically acceptable compounds associated with the synthesis of polyamines, or mixtures thereof (component (c)); and
(d) a nucleobase source (component (d)),

wherein component (a) and optionally component (c) are the only amino acids in the medicament of nutritional formulation which are added as free amino acids or in physiologically acceptable salt form.

Said medicaments and nutritional formulations are hereinafter designated "diets of the inventions".

The dosage should be such that the medicaments or nutrional formulations are effective for the diminution of tumour necrosis factor (TNF) levels in patients in whom said levels are elevated beyond those which mediate physiological homeostasis and local inflammation.

One unit dose of such a medicament or nutritional formulation preferably comprises 1.5 to 80 parts by weight of component (a) in association with the following amounts of one or more components selected from (b) to (d): 0.1 to 20 parts by weight of component (b), 3 to 40 parts by weight of component (c) and 0.1 to 4.0 parts by weight of component (d). Particularly preferred one unit dose comprises 1.5 to 80 parts by weight of component (a) in association with the following amounts of one or more components selected from (b) to (d): 2 to 5 parts by weight of component (b), 7.5 to 20 parts by weight of component (c) and 1.7 to 2.0 parts by weight of component (d).

The amount of components (a) to (d) administered daily will conveniently correspond to 1.5 to 80 g for component (a), 0.1 to 20 g, preferably 2 to 5 g, for component (b), 3 to 40 g, preferably 7.5 to 20 g, for component (c) and 0.1 to 4.0 g, preferably 1.7 to 2.0 g, for component (d).

With respect to component (d) the above dosage is indicated for RNA, DNA, nucleosides or nucleotides. For nucleobases one weight unit of nucleobases is regarded to be equivalent to 2.5 to 3.0 weight units of RNA, DNA, nucleosides or nucleotides.

Where medicaments or nutritional formulations comprising an amino acid of the invention in combination with one or more of the above-mentioned components (b), (c) and (d) are used, such medicaments or nutritional formulations will conveniently comprise in one unit dose

(a) 1.5 to 80 parts by weight of one or more amino acids selected from the group consisting of glycine, alanine and serine, in free form or physiologically acceptable salt form, or mixtures thereof,

in combination with one or more compounds selected from the group consisting of

(b) 2 to 5 parts by weight omega-3 polyunsaturated fatty acids;
(c) 7.5 to 20 parts by weight L-arginine or L-ornithine, or mixtures thereof; and
(d) 1.7 to 2.0 parts by weight RNA.

Preferred medicaments or nutritional formulations comprise in one unit dose:

(a) from 1.5 to 80 parts by weight of an amino acid selected from the group consisting of glycine, alanine and serine, in free form or physiologically acceptable salt form, or mixtures thereof, in association with
(c) 3 to 40 parts by weight, preferably 7.5 to 20 parts by weight, of arginine or an equivalent amount of one or more other physiologically acceptable compounds associated with the synthesis of polyamines, or an equivalent amount of a mixture of arginine with such compounds.

More preferably the medicaments or nutritional formulations of the invention comprise component (a) in combination with component (c) at a weight ratio of 1:2 to 4:1, particularly preferred at a weight ratio of 1:1 to 2:1.
Further preferred medicaments or nutritional formulations comprise in one unit dose:

(a) from 1.5 to 80 parts by weight of an amino acid selected from the group consisting of glycine, alanine and serine, in free form or physiologically acceptable salt form, or mixtures thereof, in association with
(b) 0.1 to 20 parts by weight, preferably 2 to 5 parts by weight, of omega-3 PUFAs; and
(c) 3 to 40 parts by weight, preferably 7.5 to 20 parts by weight, of arginine or an equivalent amount of one or more other physiologically acceptable compounds associated with the synthesis of polyamines, or an equivalent amount of a mixture of arginine with such compounds.

Omega-3 PUFAs are conveniently protected against peroxidation.
Physiologically acceptable ways of protecting omega-3 PUFAs against peroxidation are known in the art. They include physiologically acceptable micro-encapsulation of omega-3 PUFAs and the use of physiologically acceptable antioxidants.
A typical example suitable for use as physiologically acceptable micro-encapsulation agents is starch. The micro-encapsulation can be effected in a manner known <u>per se.</u> The micro-encapsules may be coated in a manner known <u>per se</u>, by physiologically acceptable coating agents such as Gum Arabic.
Typical examples of antioxidants suitable for use in the method of the invention include antioxidant vitamins such as Vitamin C, Vitamin E or mixtures thereof.
The amount of antioxydant added should be sufficient to prevent peroxidation of the omega-3 PUFAs. Such amounts can be easily calculated. In general, for convenience, any antioxydants employed to prevent peroxidation, will be employed in excess. It will be appreciated that the presence of any other agent administered in association with the omega-3 PUFAs may require adjustment of the amount of antioxidant to be employed.
The omega-3 PUFAs may be employed in a form suitable for the physiological supply of omega-3 PUFAs, e. g. in free acid form, in triglyceride form, or in the form of physiologically acceptable natural sources of omega-3 PUFAs. Such natural sources include linseed oil and fish oils such as menhaden oil, salmon oil, mackerel oil, tuna oil, codliver oil and anchovy oil. Said natural sources, in particular, the fish oils, comprise substantial amounts of omega-3 fatty acids. Where the omega-3 PUFAs are employed in triglyceride form, said triglycerides may comprise esters with other physiologically acceptable fatty acids. Preferred omega-3 PUFAs include eicosapentaenoic acid (EPA) and docosahexaenoic acid (DHA), in free acid form, in triglyceride form or in form of natural sources having a high EPA and/or DHA content.
When the amino acids of the invention are administered in the form of a medicament such a medicament will comprise from 1 to 99 g of the amino acid of the invention per 100g.
In general, favourable effects are obtained when administering the diets of the invention in the form of a formula diet, which may, depending on the circumstances be a complete formula diet (i.e. a diet supplying essentially all required energy, amino acids, vitamins, minerals and trace elements) or a diet supplement. The diet will conveniently be taken in aqueous liquid form. A formula diet accordingly may comprise a source of carbohydrates, lipids fat (fat source) and protein (nitrogen source), and at least one amino acid selected from the group consisting of glycine, L-alanine and L-serine, or physiologically acceptable salts thereof, characterized in that the acid or salt is present in the formula diet in an amount of about 0.5 to 10g per 100g. The formula diet will preferably further comprise other nutritionally advantageous components such as vitamins, minerals, trace elements, fibers (preferably soluble fibers).

Examples of suitable nitrogen sources include nutritionally acceptable proteins such as soy bean or whey derived proteins, caseinates, and/or protein hydrolysates. Suitable carbohydrate sources include sugars such as maltodextrins. Examples of suitable fat sources include triglycerides, as well as di- and monoglycerides.

Examples of vitamins suitable for incorporation into the medicament or formulation of the invention include Vitamin E, Vitamin A, Vitamin D, Vitamin K, folic acid, thiamin, riboflavin, Vitamin $B_1$, $B_2$, $B_6$ and $B_{12}$, niacin, biotin and panthotenic acid in nutritionally acceptable form.

Examples of mineral elements and trace elements suitable for incorporation into the medicament or formulation include sodium, potassium, calcium, phosphorous, magnesium, manganese, copper, zinc, iron, selenium, chromium, and molybdenum in nutritionally acceptable form.

In particular, the medicament or formulation will preferably comprise beta-carotene (Vitamin A), Vitamin E, Vitamin C, thiamine, Vitamin $B_{12}$, choline, selenium and zinc in nutritionally acceptable form.

The term "soluble fiber" as used herein refers to fibers which are able to undergo substantial fermentation in the colon ultimately to produce short chain fatty acids. Examples of suitable soluble fibers include pectin, guar gum, locust bean gum, xanthan gum which may optionally be hydrolysed. For adults the total amount of soluble fibre per day will conveniently lie in the range of from 3 to 30g.

It will be appreciated that omega-3 PUFAs may be administered in higher amounts than those indicated hereinabove, and that such higher amounts will in general not impair the desired effect or provoke undesired side effects.

Compounds particularly suitable for use as component (c) in the formulation of the invention include L-arginine and L-ornithine, most preferably L-arginine. Component (c) may be employed in free form, physiologically acceptable salt form, e.g. in the form of a salt with phosphoric acid, citric acid, tartaric acid, fumaric acid, adipic acid or lactic acid, or in small peptide form. Preferably L-arginine in free form is employed.

The term small peptides as used herein refers to peptides having from 2 to 6, preferably from 2 to 4 amino acids.

As already indicated, omega-3 PUFAs will conveniently be administered in the form of fish oils, protected or not against peroxidation. Such fish oils also comprises omega-6 PUFAs.

Omega-6 PUFAs have also a favourable effect on the immune response and on the resistance to infection upon surgery. Accordingly, diets of the invention will conveniently further comprise omega-6 PUFAs.

For the purpose of the invention the omega-6 PUFAs may be in free acid form or in a form suitable for the physiological supply of omega-6 PUFAs, e.g. in triglyceride form. Examples of omega-6 PUFAs particularly appropriate for use according to the invention, include linoleic acid and arachidonic acid, linoleic acid being most preferred. Examples of suitable omega-6 PUFA sources are known in the art. They include fish oils and vegetable oils. Examples of omega-6 PUFA sources having a high linoleic acid content such as safflower oil, sunflower oil, soya oil, cotton oil and corn oil.

Administration of a daily amount of omega-6 PUFAs in the range of from 1.5 to 5.0 g will in general suffice to attain a favourable effect. One unit dose of the medicaments or nutritional formulation defined above may accordingly further contain 1.5 to 5 parts by weight of omega-6 PUFAs.

In addition to components (b), (c) and (d), and omega-6 PUFAs further components may be added to the diets of the invention and may have a beneficial effect on the activity of the amino acid of the invention. An example of such beneficial components are omega-9 PUFAs. A preferred natural source for such fatty acid mixtures are fish oils. For taste and other reasons, the fish oils will, in oral application forms, preferably be used in encapsulated form.

Where the formula diet of the invention is intended for use as a nutritional supplement (e.g. pre-operative treatment), the amount of energy supplied by it should not be too excessive, in order not to unnecessarily suppress the patients appetite. The supplement should conveniently comprise energy sources in an amount supplying from 600 to 1000 Kcal/day. For use as a complete formula diet (e.g. for post-operative treatment, treatment of trauma), the diets of the invention will conveniently supply from 600 to 1500 Kcal/day. The contribution of the nitrogen source, carbohydrate source and lipid source to the total daily caloric may vary within wide ranges. In preferred formulations of the invention the carbohydrate source provides for 40 to 70 % of the total energy supply and, the nitrogen and fatty acid source each for 15 to 30 % of the total energy supply of the formulation. For use as complete diet, the diet of the invention will conveniently be administered in aqueous liquid form in volumes in the range of from 500 ml to 3000 ml. For use as a supplement, the administration may be in powder or liquid form.

Conditions or diseases in which patients have elevated TNF levels which can be treated in accordance with the invention include the following: osteoporosis; arthritis; metastasis; lung diseases such as adult respiratory distress syndrome (ARDS); inflammatory bowel diseases such as ulcerative colitis and Crohn's disease; coronary artery disease; liver cirrhosis - whether alcohol or viral induced; sepsis, including endotoxic shock; hypoxia reperfusion injury; the wasting syndrome observed in AIDS and cancer patients; hepatitis; nephritis; pathological angiogenesis; treatments such as radiation and chemotherapy which - for example - prejudice bone marrow; organ rejection; trauma; post-ischemic heart injury; ulcers; degenerative conditions such as motor neurone disease and multiple sclerosis which are associated with the nervous system; brain injury and inflammation; pancreatitis; renal failure; diabetes; stroke; asthma and infection. Especially good results are achieveable in the treatment of sepsis, endotoxic shock, infection and hypoxia reperfusion injury, particularly of endotoxic shock and sepsis. Also particularly good results are achievable in the treatment of inflammatory bowel diseases, sepsis, chronic liver diseases, pathological angiogenesis, arteriosclerosis and trauma.

Infections may be wound infections, empyemas, bacteremias, abscesses, septicemias and the like. They may be caused by a variety of infectious agents, including bacteria, viruses, parasites, fungi and endotoxins.

Generally speaking, the treatment with the amino acid of the invention is indicated for patients in whom TNF levels could be/are at least transiently elevated above those levels which mediate physiological homeostasis and local inflammation. Such patients include e.g. trauma patients (polytrauma, burns, major surgery); patients with systemic inflammatory response syndrome (SIRS); septic patients; adult respiratory distress syndrome (ARDS) patients; patients with acute liver failure in whom no pretreatment is possible but an acute effect is desired; patients at infection risk such as patients having a lowered resistance due to immunosuppression, patients subject to radio- and/or chemotherapy, patients suffering from diabetes mellitus, from protein-malnourishment, gastrointestinal cancer surgery patients, cardiac surgery patients, patients subject to transplantations, patients having an increased risk of liver disease due to excessive alcohol consumption and patients suffering from human immunodeficiency virus-related infection; and patients before and following major operative procedures, i.e. any operative procedure requiring general anesthesia such as cardiac bypass surgery and major upper gastrointestinal surgery.

Administration of a medicament or nutritional formulation containing an amino acid of the invention in combination with component (c) is particularly indicated for the treatment of osteoporosis.

The surprising pharmacological activity of glycine, or the amino acid of the invention respectively, of diminishing TNF levels in patients in whom said levels are elevated beyond those which mediate physiological homeostasis and local inflammation is useful, in that it allows i.a. to minimize the effects of endotoxemia, hypoxia-reperfusion injury and infection and to reduce the risks of endotoxic shock and sepsis, and thus reduce or prevent the risk of mortality.

The amino acids of the invention and especially the diets of the invention are as already set out above particularly suitable for treatment of patients due for surgery. Such pretreatment will be most effective when administering the diet of the invention in the form of a supplement.

The supplement will advantageously be administered over a period of 3 days or longer. In general, a pretreatment starting 3 to 6 days before surgery, and during said 3-6 day period will be sufficient to attain the desired effect. For pretreatment or prophylactic purposes administration of a supplement containing from 1.5 g to 80 g amino acid of the invention in association with from 2 to 5 g Component (b) (omega-3-PUFAs) and/or with Component (c) supplying from 7.5 to 20 g L-arginine or L-ornithine per day, will in general give the desired effect.

Such a supplement may and preferably will also contain an effective amount of component (d), omega-6 PUFAs or further components as set out above.

The supplement will conveniently be administered in the form of unit doses suitable for administration of the supplement 3 to 4 times per day. Where the diets of the invention comprise energy sources, it is appropriate not to supply more than 1500 Kcal/day. Apart from this limitation with respect to the energy supply, diet supplements of the invention for diminuition of TNF levels can and will conveniently be supplied in the form of complete formula diets as described above.

Where acute treatment of patients following excessive ethanol exposure is necessary, the amino acid of the invention will conveniently be administered parenterally. Typical administration forms suitable for such acute treatment are e.g. the aqueous solutions disclosed hereinbelow.

Where it is desired to minimize alcohol uptake into the blood by glycine induced alcohol elimination in the stomach, the amino acid of the invention will conveniently be provided in a conventional oral administration form, such as granules, tablets, capsules, liquids (including soups and drinks such as soft drinks, thirst quenchers), powders, formula diets etc. When formulated in a physiologically acceptable formulation form such as a capsule or tablet form, such formulations will conveniently contain 0.2 to 90 % by weight, preferably from 30 to 50 % by weight of an amino acid of the invention. In general, satisfactory alcohol (ethanol) elimination from the stomach is obtained when administering a total amount within the range of from 0.01 to 5.0 g of one or more of the amino acids selected from the group consisting of glycine, alanine and serine, in free form and/or physiologically acceptable salt form per kg body weight. The administration is conveniently orally, and prior to alcohol intake.

Typical pharmacologically acceptable formulation forms for oral administration will further comprise pharmacologically acceptable diluents, carriers, vitamins, spices, pigments and/or other adjuvants well known to the skilled person to be suitable for incorporation into such formulation.

The diets and formulations of the invention may be obtained in a manner known per se, e.g. by admixing the ingredients.

Typical formulations suitable for use according to the invention and in particular for treatment of patients having increased risk of liver diseases due to excessive alcohol consumption include aqueous solutions consisting essentially of 0.1 % to 90 % by weight of at least one amino acid selected from the group consisting of glycine, alanine and serine, and pharmaceutically acceptable salts thereof, the balance being distilled water. The amino acid of the invention may be present in a concentrated form of the solution in an amount of from 15 to 90% (by weight of the solution). Concentrated solutions are suitable for dilution to application forms or for use in acute treatment. Application forms having a lower content (e.g. 0.1 to 5 %) of the amino acid of the invention will in general be indicated for prophylactic purposes; concentrated forms of the solution having a higher content (e.g. 5 % to 40 % by weight) of amino acid of the invention will in general be more suitable for acute treatment.

Other formulations suitable for inclusion in the medicament or formulation of the invention, in particular for parenteral application, include infusion solutions such as Ringer's injection solution, lactated Ringer's injection solution, crystalloids, colloids or other plasma substitutes, in association or enriched with about 0.1 to 5.0g per liter infusion solution of glycine, serine and/or alanine. Ringer's injection solution is a sterile solution, containing from 3.23 to 3.54g of sodium (equivalent to from 8.2 to 9.0g of sodium chloride), from 0.149 to 0.165 of potassium (equivalent to from 0.285 to 0.315g of potassium chloride), from 0.082 to 0.098g of calcium (equivalent to from 0.3 to 0.36g of calcium chloride, in the form of $CaCl_2 \cdot 2H_2O$), from 5.23 to 5.80g of chloride (as NaCl, KCl and $CaCl_2 \cdot 2H_2O$) and water in sufficient quantity to give 1000 ml solution. Lactated Ringer's Injection solution is a sterile solution containing from 2.85g to 3.15g sodium, as chloride and lactate), from 0.141 to 0.173g of potassium (equivalent to from 0.27g to 0.33g of potassium chloride), from 0.049 to 0.060g calcium (equivalent to from 0.18g to 0.22g of $CaCl_2.2H_2O$), from 2.31g to 2.61g of lactate, from 3.68 to 4.08g of chloride (as NaCl, KCl and $CaCl_2 \cdot 2H_2O$) and water in sufficient quantity to give 1000 ml solution.

The terms crystalloids and colloids in connection with fluid therapy are known in the art. They include plasma substitutes such as Haemaccel (polygeline based) and Gelofusine (gelatin based).

The invention will be further understood by reference to the following specific description.

### EXAMPLE 1. Effects of dietary glycine on survival and liver injury in the rat

Male Sprague-Dawley rats (200-250 g) are fed, *ad libitum,* by powder diet containing 20% by weight of casein (control diet) or 5% by weight of glycine and 15% by weight of casein (glycine diet) for 3 days prior to injection with LPS. The rats are then injected with lipopolysaccharide (LPS; 10, 20 and 30 mg/kg resp.) *via* the tail vein and mortality is assessed 24 hours after the injection. If the rats survive 24 hours, they are considered safe as no late mortality is observed.

### RESULTS

a) The 5% glycine diet offers a 100 % protection against an LPS dose of 10 mg/kg compared to 50% mortality in the control group significant at the p<0.05 level. At an LPS dose of 20 mg/kg a 30% mortality is observed with 5% glycine diet and a 70% mortality with the control diet. At 30 mg/kg LPS mortality increased to 90% in the glycine treated animals and to 100% in the controls.

b) From the survivors, the liver enzyme AST (Aspartate Aminotransferase; a transaminase) is measured as an indication of liver damage. The AST level is markedly and significantly reduced in the glycine fed rats (from 2000IU/L in untreated fed rats treated with an LPS dose of 10 mg/kg).

c) In a parallel experimental series, the effect of glycine diet on tumor necrosis factor (TNF) is assessed. Serum TNF was measured by ELISA. After injection of 10 mg/kg LPS, serum TNF increased rapidly in rats fed with the control diet to values over 6000 pg/ml, 60 min after injection, before declining to control values. This increase is significantly suppressed (by at least 50%; p< 0.05) in rats fed with the 5% glycine diet, whereby the peak is attained 150 min after injection.

d) Liver and lung specimens for histology are taken 24hr after injection of LPS (10 mg/kg) and hematoxylin-eosin stained. Rats fed with the control diet show many necrotic areas and neutrophil infiltration in the liver and marked interstitial edema with neutrophil infiltration in the lung. Rats fed with glycine diet show less necrosis in the liver and less pulmonary edema than rats fed with the control diet.

e) Serum glycine concentration is determined by HPLC from 2 groups of rats fed with control diet and glycine diet resp., and of which each group had been given a LPS injection (10 mg/kg i.v. in the tail vein). A rat fed with glycine diet without LPS treatment shows a remarkably higher glycine concentration (1892μM) than rats fed with control diet (150μM) and without LPS injection. The rats fed with glycine diet maintain the high concentration of glycine (1727 ± 515 μM) 6 hr after LPS injection; the rats fed with control diet have 6 hr after the LPS injection a serum glycine concentration of 278μM.

**EXAMPLE 2. Effects of glycine on reperfusion injury in a low flow-reflow liver perfusion model.**

## METHODS

**Animals used**. Male Sprague-Dawley rats weighing between 180-210 g and fed a Purina diet. Rats are fasted for 24 h prior to surgery.

**Liver Perfusion.** Rats are anesthetized with pentobarbital sodium (50 mg) before surgery and livers are removed surgically and perfused *via* a cannula inserted into the portal vein with Krebs-Henseleit bicarbonate buffer (pH 7.4, 37°C) saturated with an oxygen-carbon dioxide (95:5) mixture in a non-recirculating system (Krebs-Henseleit, 1932). After surgery, livers are perfused at flow rates around 1 ml/g/min for 75 minutes (low-flow). Subsequently, livers are perfused at normal flow rates (4 ml/g/min) for 40 minutes (reflow). Glycine is dissolved in Krebs-Henseleit bicarbonate buffer (pH 7.4, 37°C) and infused into the liver continuously beginning 10 minutes before reflow at rates resulting in final concentrations ranging from 0.06-2mM.

**Lactate dehydrogenase (LDH).** LDH activity in the perfusate is determined using standard enzymic techniques (Bergmeyer, 1988). Three ml of perfusate are mixed thoroughly with a reagent containing 15% trichloroacetic acid, 0.375% thiobarbituric acid and 0.25N hydrochloric acid and heated for 15 minutes in a boiling water bath. After cooling, samples were centrifuged at 1000g for 10 minutes and the absorbance of the supernatant is determined at 535nm. Rates of release of LDH are expressed per gram wet weight of liver per hour.

**Trypan Blue Distribution Time and Histological Procedures.** To assess microcirculation and cell death in the liver, trypan blue is infused into the liver at the end of all experiments at final concentrations of 0.2 mM (Belinsky et al., 1984). The time for the liver surface to turn evenly dark blue is recorded. Excess dye is removed by perfusion with Krebs-Henseleit buffer for an additional 10 minutes. Subsequently, livers are perfused with 1% paraformaldehyde for 10 minutes and fixed tissue is embedded in paraffin and processed for light microscopy. Sections are stained only with eosin, a cytoplasmic stain, so that trypan blue can be identified readily in the nuclei of damaged cells.

All nuclei of parenchymal cells in a zone radiating five cells from either periportal or pericentral regions are identified as trypan blue positive or negative. The percentage of staining is calculated from the number of stained nuclei divided by the total number of cells in any given region.

**Statistical Analysis.** Student's-t-test or ANOVA was used where appropriate. Differences are considered significant when the p-value is less than 0.05.

## RESULTS

**Effects of Glycine on Hepatocellular Damage in a Low-flow, Re-flow Perfusion Model.** During the low-flow period, LDH release is minimal (around 1 IU/g/h at 75 minutes). When the flow rate is increased to 4 ml/g/min, however, LDH release increases gradually, reaching a new steady-state value in about 30 minutes. Maximal LDH release during the reperfusion period is around 35 IU/g/h in controls, but is reduced significantly by glycine treatment in a dose-dependent manner. When the concentration of glycine is increased to 2mM, LDH release is reduced to around 5 IU/g/h; half-maximal decreases occur with 180μM glycine.

Trypan blue uptake indicates irreversible loss of cell viability in the liver lobule. Reflow for 40 minutes following 75 minutes of low-flow hypoxia causes death in about 30% of parenchymal cells in pericentral regions, but only affected about 2% of cells in previously normoxic periportal regions. Infusion of glycine (2mM) decreases cell death in pericentral areas to 9 %. Taken together, reperfusion injury, which occurs when oxygen is re-introduced into previously anoxic pericentral regions of the liver lobules, is clearly reduced by acute glycine infusion in a dose-dependent manner.

**Effects of Glycine on Trypan Blue Distribution.** Trypan blue distribution time, an indicator of the hepatic microcirculation, is slightly but significantly lower in glycine-infused livers than in controls (about 190 seconds in glycine-treated liver and 225 seconds in controls, respectively, p<0.05, n=5) when trypan blue is infused into the liver 5 minutes after reperfusion. However, values are reduced dramatically by glycine in a dose-dependent manner when trypan blue is infused at the end of 40 minutes of reperfusion. It took about 460 seconds for trypan blue to distribute evenly in controls, whereas values are reduced to about 250 seconds when 2 mM glycine is infused. The concentration which caused half-maximal decrease in trypan blue distribution time is also around 180 μM.

Glycine minimizes LDH release and cell death almost completely during reperfusion in a dose-dependent manner. Glycine has accordingly potent cytoprotective effects against reperfusion injury in a low-flow, reflow liver perfusion model in the rat.

Trypan blue distribution time is reduced by glycine in a dose-dependent manner, with the half-maximal effect similar to the cytoprotective effect of glycine (180µM). Since trypan blue distribution time can be influenced not only by disturbances of the hepatic microcirculation but also by hepatic cell injury, trypan blue distribution time is measured after only 5 minutes of reflow, when reperfusion injury is minimal. This value is also reduced significantly by glycine but to a smaller extent, indicative of improved hepatic microcirculation.

Other possible mechanisms related to anoxia and reperfusion injury such as ATP depletion and alteration of mitochondrial function are also investigated. Bile production, a highly energy-dependent process, is not affected by glycine, indicating that glycine does not minimize ATP depletion, and oxygen uptake, an indicator of mitochondrial function, is not altered by glycine either. In conclusion, glycine improves hepatic microcirculation and protects against oxygen-dependent reperfusion injury.

## EXAMPLE 3. Effect of pre-treatment with glycine on the mortality after partial hepatic ischemia/reperfusion and LPS injection

After 3 days feeding with control diet and glycine diet resp., rats are given partial hepatic ischemia for 90 min. under methoxyflurane anesthesia. A sublethal dose of LPS (5 mg/kg) is injected via the tail vein 6 hr after reperfusion.

## RESULTS

All of the control rats without LPS injection survived for 24 hours after 90 min. partial hepatic ischemia/reperfusion (n=4). All rats fed with control diet and with 90 min. partial hepatic ischemia/reperfusion die within 24hr after the (sublethal) LPS injection (n=4).

Pretreatment of rats with glycine diet markedly improved the survival (5 rats out of 6 survived) under the same conditions (90 min. partial hepatic ischemia/reperfusion and given the LPS injection) during the observation period (24 hours from the injection time); $p < 0.05$ with Fisher's test.

## Example 4: Effects of dietary glycine on alcohol-induced liver injury

### 4.1 Materials and Methods

#### a. Animals

In Male Wistar rats, weighing 300 to 320 mg each, intragastric cannulas were inserted as described by Tsukamoto and French. Cannulas were tunnelled subcutaneously to the dorsal aspect of the neck and attached to infusion pumps by means of a spring-tether device and swivel allowing complete mobility of rats in metabolic cages. Animals were infused continuously with a high-fat liquid diet containing ethanol for up to 4 weeks. All animals received humane care in compliance with institutional guidelines.

#### b. Diet

A liquid diet described by Thompson and Reitz was used. It contained corn oil as fat (37 % of total calories), protein (23 %), carbohydrate (5 %), minerals and vitamins, plus ethanol or isocaloric maltose-dextrin (35 %), hereinafter designated liquid control diet. Glycine (2 or 5 % by weight) was added to the liquid control diet; such diets are hereinafter referred to as liquid 2 % glycine diet and liquid 5 % glycine diet resp.

#### c. Urine collection and assay of ethanol

Concentration of ethanol in urine, which are representative of blood alcohol levels were measured daily. Rats were housed in metabolic cages that separated urine from feces, and urine was collected over 24 hours in bottles containing mineral oil to prevent evaporation. Each day at 0900h, urine collection bottles were changed and an 1-ml sample was stored at -20° C in a microtube for later ethanol analysis. Ethanol concentration was determined by measuring absorbance at 360 nm resulting from the reduction of $NAD^+$ to NADH by alcohol dehydrogenase.

#### d. Blood collection and asparate aminotransferase (AST)

Blood was collected via the tail vein once a week and centrifuged. Serum was stored at -20° C in a microtube until assayed for AST by standard enzymatic procedures. Whole blood (100 µl) was also assayed for ethanol as described below, and hepatic portal blood was also collected when the liver biopsy were performed at the 2nd and 4th week of treatment with ethanol.

**e. Ethanol assay in breath, peripheral and portal blood, feces and stomach contents**

To determine concentrations of ethanol in breath, rats were forced to breathe into a closed heated chamber (37° C) for 20 sec. and 1 ml of breath was collected with a gas-tight syringe. Ethanol concentrations were analysed by gas chromatography (GC). Ethanol in peripheral and portal blood was also assayed by GC. Blood (100 µl) was mixed with 900 µl of distilled water in a closed flask incubated for 30 min. at 37° C, and 1 ml of the gas phase was collected and assayed by GC. Rat feces were collected directly from the anus, homogenized in distilled water and incubated and analysed as described above for blood. Stomach contents were also collected when rats were sacrificed at the 4th week of ethanol treatment, and analysed as described for blood.

**f. Measurement of glycine concentration in blood**

After 4 weeks of ethanol treatment, 500 µl of plasma was collected and stored at - 80° C for determination of glycine concentration in blood by HPLC. Quantitative analysis of glycine in heparinized plasma was carried out using the PICP-TAG (Waters, Milford, MA) method. Plasma samples were first hydrolysed with HCl, and then derivatized with phenylisothiocyanate (PITC) to produce phenylthiocarbamyl (PTC) amino acids. Amino acids including glycine were determined by automated gradient reserve phase high-pressure liquid chromatography (HPLC).

**g. Pathological evaluation**

Rats underwent liver biopsy and autopsy after 2 and 4 weeks of treatment with ethanol. Livers were formalin-fixed, embedded in paraffin and stained with hematoxylin and eosin to assess steatosis, inflammation and necrosis. Liver pathology was scored as described by Nanji et al. as follows: steatosis (the percent liver cells containing fat): <25 % = 1 +; <50 % = 2+; <75 % = 3 +, > 75 % = 4+; inflammation and necrosis: 1 focus per low-power field = 1+; 2 or more = 2+.

**h. Statistics**

ANOVA or Student's t-test was used for determination of statistical significance as appropriate. For comparison of pathological scores, the Kruskal-Wallis ANOVA for ranks was used. A p value less than 0.05 was selected before the study as the level of significance.

4.2 RESULTS

Body weights of rats fed with the liquid control diet, the liquid/glycine diet and the liquid 5 % glycine diet, during the course of this study had a tendency to decline during the first week was observed. The body weights then stabilized and were constant during the following 3 weeks of treatment with ethanol. There were no significant differences in body weight among the groups studied.

Ethanol intake was gradually increased to 9 to 10 gm/kg/day during the first week after surgery. Intake was between 10 and 13 gm/kg/day during weeks 2-4, and there were no significant differences between the groups. Glycine concentration in plasma after 4 weeks of treatment was 779 $\pm$ 66 µmol/L in rats receiving the liquid 5 % glycine-containing diet, and was 198 $\pm$ 16 µmol/L in ethanol fed rats.

Representative plots of daily urine alcohol concentrations in rats fed with the liquid control diet and the liquid glycine diets were determined. Alcohol levels fluctuate in a cyclic pattern from near zero to greater than 300 mg/dl in rats fed with the liquid control diet, even though ethanol was infused continuously. In rats fed with one of the liquid glycine diets, alcohol concentrations were very low but were still cyclic. Mean urine alcohol concentrations were reduced significantly in a dose-dependent manner; a 50 % reduction of the mean urine alcohol concentration was obtained by feeding with the liquid 2 % glycine diet, a 70 % reduction with the liquid 5 % glycine diet.

Serum AST in rats fed with the liquid control diet increased gradually with time of exposure and reached a level of 183 IU/L after 4 weeks (control value 70 IU/L). This increase was attenuated significantly by both glycine treatments at every point during the study. After 4 weeks of treatment, serum AST level was 66 IU/L in 2 % glycine-treated rats fed with the liquid 2 % glycine diet and was 100' IU/L in the group fed with the liquid 5 % glycine diet.

In rats fed with the liquid control diet, slight steatosis was observed after only 2 weeks of treatment. After 4 weeks of treatment with said liquid control diet, obvious fatty changes were not apparent in control rats, not treated with ethanol. In rats fed with liquid 2 % glycine and liquid 5 % glycine diet, fatty changes were attenuated and necrosis and inflammation were almost totally absent. The reductions of steatosis and necrosis were statistically significantly after feeding with the liquid glycine diets; only a tendency for reduction of inflammation was observed after feeding with the liquid glycine diets because of variability.

Eventually, alcohol in stomach contents in glycine-treated rats was also reduced dramatically. Therefore, it is clear that glycine reduces the ethanol concentration in the stomach, possibly by effects on ethanol metabolism.

## TABLE 1

## Effect of Glycine on ethanol concentrations in rats on the Tsukamoto-French Model

| Treatment | Breath | Feces | U r i n e | | Blood | Stomach | |
|---|---|---|---|---|---|---|---|
| | | | A.M. | 24 hr | Tail | Portal Vein | |
| Ethanol | 108 ± 12 | 82 ± 21 | 184 ± 12 | 211 ± 16 | 199 ± 45 | 357 ± 65 | 761 ± 625 |
| Ethanol + 5 % Glycine | 5 ± 4*** | 15 ± 11* | 26 ± 25** | 24 ± 8*** | 21 ± 13** | 10 ± 7*** | 26 ± 41* |

Breath, feces, blood and stomach contents were collected from rats and analysed by headspace gas chromatography. Urine samples were analysed enzymatically. Samples are taken from a total of 6 rats. Results are expressed as Mean ± S.E.M.

* p < 0.05; ** p< 0.01; ***p < 0.001 for comparisons of ethanol vs. ethanol + glycine-fed rats values by Student's t-test.

## EXAMPLE 5: ENTERAL COMPOSITIONS

In the following compositions MM stands for "mineral mixture", SM for "trace element mixture" and VM for "vitamin mixture". The composition of these three mixtures is as follows:

| MM | |
|---|---|
| Ingredients | g/100g |
| Maltodextrins | 34.40 |
| Potassium citrate/phosphate | 34.60 |
| Magnesium dicitrate | 8.20 |
| Calcium chloride | 8.00 |
| Sodium citrate/chloride | 9.00 |
| Citric acid | 3.50 |
| Choline tartrate | 2.30 |

| SM | |
|---|---|
| Ingredients | g/100g |
| Maltodextrins | 47.79 |
| Molybdenum-yeast | 18.00 |
| Chromium-yeast | 9.20 |
| Zinc sulfate | 7.00 |
| Selenium-yeast | 7.00 |
| Ferrum(II) sulfate | 6.92 |
| Copper(II) gluconate | 2.24 |
| Manganese(II) sulfate | 1.12 |
| Sodium fluoride | 0.70 |
| Potassium iodide | 0.03 |

| VM | |
|---|---|
| Ingredients | g/100g |
| Maltodextrins | 43.44 |
| Sodium ascorbate | 35.00 |
| Vitamin E-Ac. 50% | 16.00 |
| Niacinamide | 1.55 |
| Vitamin A-Acetate | 1.20 |
| Ca-D-Panthothenat | 0.98 |
| Vitamin $K_1$ 1% | 0.71 |
| Vitamin $B_{12}$ 0.1% | 0.30 |
| Vitamin $D_3$ | 0.28 |
| Vitamin $B_6$ | 0.20 |
| Vitamin $B_1$ | 0.17 |
| Vitamin $B_2$ | 0.15 |
| Folic acid | 0.02 |
| Biotin | 0.01 |

| Composition Comprising Glycine | |
|---|---|
| Ingredients | g/100g |
| Water | 77.40 |
| Maltodextrins | 10.10 |
| Na/Ca caseinates | 4.60 |
| Glycine | 3.00 |
| MM | 2.00 |
| SM | 0.05 |
| VM | 0.10 |
| β-Carotine | 0.03 |
| Lipids: | |
| Palm oil | 2.33 |
| Sunflower oil | 0.26 |
| Emulsifier Nathin E | 0.13 |
| | 100.00 |

| Composition Comprising Glycine and Arginine | |
|---|---|
| Ingredients | g/100g |
| Water | 77.40 |
| Maltodextrins | 8.93 |
| Na/Ca caseinates | 4.60 |
| Glycine | 3.00 |
| Arginine | 1.17 |
| MM | 2.00 |
| SM | 0.05 |
| VM | 0.10 |
| β-Carotine | 0.03 |
| Lipids: | |
| Palm oil | 2.36 |
| Sunflower oil | 0.23 |
| Emulsifier Nathin E | 0.13 |
| | 100.00 |

| Composition Comprising Glycine and Fish Oil (ω-3 fatty acids) | |
|---|---|
| Ingredients | g/100g |
| Water | 77.40 |
| Maltodextrins | 10.10 |
| Na/Ca caseinates | 4.60 |
| Glycine | 3.00 |
| MM | 2.00 |
| SM | 0.05 |
| VM | 0.10 |
| β-Carotine | 0.03 |
| Lipids: | |
| Palm oil | 1.32 |

(continued)

| Composition Comprising Glycine and Fish Oil ($\omega$-3 fatty acids) | |
| --- | --- |
| Ingredients | g/100g |
| Lipids: | |
| Sunflower oil | 0.23 |
| Emulsifier Nathin E | 0.13 |
| Fish Oil EPAX 3000 TG | 1.04 |
| | 100.00 |

| Composition Comprising Glycine and RNA | |
| --- | --- |
| Ingredients | g/100g |
| Water | 77.40 |
| Maltodextrins | 9.96 |
| Na/Ca caseinates | 4.60 |
| Glycine | 3.00 |
| Yeast extract RNA | 0.14 |
| MM | 2.00 |
| SM | 0.05 |
| VM | 0.10 |
| $\beta$-Carotine | 0.03 |
| Palm oil | 2.33 |
| Sunflower oil | 0.26 |
| Emulsifier Nathin E | 0.13 |
| | 100.00 |

| Composition Comprising Glycine, Arginine and Fish Oil ($\omega$-3 fatty acids) | |
| --- | --- |
| Ingredients | g/100g |
| Water | 77.40 |
| Maltodextrins | 8.93 |
| Na/Ca caseinates | 4.60 |
| Glycine | 3.00 |
| Arginine | 1.17 |
| MM | 2.00 |
| SM | 0.05 |
| VM | 0.10 |
| $\beta$-Carotine | 0.03 |
| Lipids: | |
| Palm oil | 1.32 |
| Sunflower oil | 0.23 |
| Emulsifier Nathin E | 0.13 |
| Fish Oil EPAX 3000 TG | 1.04 |
| | 100.00 |

| Composition Comprising Glycine, Arginine and RNA | |
| --- | --- |
| Ingredients | g/100g |
| Water | 77.40 |
| Maltodextrins | 8.79 |
| Na/Ca caseinates | 4.60 |
| Glycine | 3.00 |
| Arginine | 1.17 |
| Yeast extract RNA | 0.14 |
| MM | 2.00 |
| SM | 0.05 |
| VM | 0.10 |
| β-Carotine | 0.03 |
| Lipids: | |
| Palm oil | 2.33 |
| Sunflower oil | 0.26 |
| Emulsifier Nathin E | 0.13 |
| | $\overline{100.00}$ |

| Composition Comprising Glycine, RNA and Fish Oil (ω-3 fatty acids) | |
| --- | --- |
| Ingredients | g/100g |
| Water | 77.40 |
| Maltodextrins | 9.96 |
| Na/Ca caseinates | 4.60 |
| Glycine | 3.00 |
| Yeast extract RNA | 0.14 |
| MM | 2.00 |
| SM | 0.05 |
| VM | 0.10 |
| β-Carotine | 0.03 |
| Lipids: | |
| Palm oil | 1.32 |
| Sunflower oil | 0.23 |
| Emulsifier Nathin E | 0.13 |
| Fish Oil EPAX 3000 TG | 1.04 |
| | $\overline{100.00}$ |

| Composition Comprising Glycine, Arginine, RNA and Fish Oil (ω-3 fatty acids) | |
| --- | --- |
| Ingredients | g/100g |
| Water | 77.40 |
| Maltodextrins | 8.79 |
| Na/Ca caseinates | 4.60 |
| Glycine | 3.00 |
| Arginine | 1.17 |
| Yeast extract RNA | 0.14 |
| MM | 2.00 |
| SM | 0.05 |

(continued)

| Composition Comprising Glycine, Arginine, RNA and Fish Oil ($\omega$-3 fatty acids) | |
|---|---|
| Ingredients | g/100g |
| VM | 0.10 |
| β-Carotine | 0.03 |
| Lipids: | |
| Palm oil | 1.32 |
| Sunflower oil | 0.23 |
| Emulsifier Nathin E | 0.13 |
| Fish Oil EPAX 3000 TG | 1.04 |
| | $\overline{100.00}$ |

As already set out above fish oil is a natural source for omega-3 PUFAs whereas sunflower oil is a natural source for omega-6 PUFAs.

Whilst the invention has been exemplified with reference to the capacity of glycine to suppress an increase in TNF and thereby minimize the effects of endotoxic shock and ischemic reperfusion injury in rats, the skilled man having had the benefit of the present disclosure will appreciate that the invention includes further aspects in addition to those specifically disclosed. For example, the invention further provides the use of at least one amino acid - or precursor thereof, selected from the group consisting of glycine, alanine and serine, or the physiologically acceptable salts thereof, to potentiate synergistically the immunosuppressive effects of cyclic polypeptide immuno-suppressants, neutralizing antibodies which have been raised against TNF or soluble TNF receptors. Such use may also provide a safening effect in respect of the immuno-suppressant, which may be cyclosporin or FK-506, for example. Moreover, patients suffering from a wide variety of diseases and/or conditions may benefit from administration to them of the medicament or formulation of the invention. Such diseases and conditions include: osteoporosis; arthritis; metastasis; ARDS/lung disease; inflammatory bowel diseases such as ulcerative colitis and Crohn's disease; coronary artery disease; liver cirrhosis - whether alcohol or viral induced; sepsis, including endotoxic shock; the wasting syndrome observed in AIDS and cancer patients; hepatitis; nephritis; pathological angiogenesis; treatments such as radiation and chemotherapy which - for example - prejudice bone marrow; organ rejection; trauma; post-ischemic heart injury; ulcers; degenerative conditions such as motor neurone disease and multiple sclerosis which are associated with the nervous system; brain injury and inflammation; pancreatitis; renal failure; diabetes; stroke and asthma.

**Claims**

1.  Use of at least one amino acid selected from the group consisting of glycine, alanine and serine, or the physiologically acceptable salts thereof, in the preparation of a medicament or nutritional formulation for enteral administration for the diminuition of tumor necrosis factor (TNF) levels in patients in whom said levels are elevated beyond those which mediate physiological homeostasis and local inflammation.

2.  The use according to claim 1, wherein such diminuition of TNF levels is achieved by inhibition or diminuition of:

    (i) Tumor necrosis factor (TNF) production by macrophage-type cells;
    (ii) the release of TNF from macrophage-type cells; and/or
    (iii) the binding of TNF by TNF receptors.

3.  The use according to claim 1 or 2 for preventing or reducing the risk of liver disease due to excessive alcohol consumption and intestinal and pancreatic disorders resulting therefrom.

4.  The use according to claim 1 or claim 2 for the prevention or diminuition of TNF-induced allograft rejection.

5.  The use according to claim 1 or claim 2 for the treatment of a disease or condition selected from the following group consisting of: osteoporosis; arthritis; metastasis; lung diseases including adult respiratory distress syndrome (ARDS); inflammatory bowel diseases including ulcerative colitis and Crohn's disease; coronary artery disease; alcohol- or viral-induced liver cirrhosis; sepsis, including endotoxic shock; hypoxia reperfusion injury; the wasting syndrome observed in AIDS and cancer patients; hepatitis; nephritis; pathological angiogenesis; post treatment syndromes following radiation or chemotherapy treatments, including prejudiced bone marrow; organ rejection; trauma; post-ischemic heart injury; ulcers; degenerative conditions such as motor neurone disease and multiple sclerosis which are associated with the nervous system; brain injury and inflammation; pancreatitis; renal failure; diabetes; stroke; asthma; infection; chronic liver diseases and arteriosclerosis.

6. The use according to claim 5 for minimizing the effects of endotoxemia, hypoxia-reperfusion injury and infection."

7. The use according to claim 6 for reducing or preventing the risk of mortality by reducing the risks of endotoxic shock and sepsis."

8. A medicament or nutritional formulation comprising effective amounts of:

    (a) one or more amino acids selected from the group consisting of glycine, alanine and serine, in free form or physiologically acceptable salt form, or mixtures thereof (component (a))
    in combination with one or more components selected from
    (b) omega-3 PUFAs where desired in admixture with omega-6 PUFAs (component (b));
    (c) L-arginine or other physiologically acceptable compounds associated with the synthesis of polyamines, or mixtures thereof (component (c));
    (d) a nucleobase source (component (d)),

    wherein component (a) and optionally component (c) are the only amino acids in the medicament or nutritional formulation which are added as free amino acids or in physiologically acceptable salt form of said amino acid.

9. The medicament or nutritional formulation according to claim 8 comprising, in one unit dose,

    (a) 1.5 to 80 parts by weight of one or more amino acids selected from the group consisting of glycine, alanine and serine, in free form or physiologically acceptable salt form, or mixtures thereof
    in combination with one or more compounds selected from
    (b) 2 to 5 parts by weight omega-3 polyunsaturated fatty acids;
    (c) 7.5 to 20 parts by weight L-arginine or L-ornithine, or mixtures thereof; and
    (d) 1.7 to 2.0 parts by weight RNA.

10. The medicament or nutritional formulation according to claim 8 or claim 9 comprising component (a) in combination with component (c).

11. The medicament or nutritional formulation according to claim 8 or claim 9 comprising component (a) in combination with components (b) and (c).

12. The medicament or nutritional formulation according to claim 8 or claim 9 comprising component (a) in combination with components (b), (c) and (d).

13. The medicament or nutritional formulation according to claim 8 which is a formula diet further comprising a source of carbohydrates, lipids fat (fat source) and protein (nitrogen source) and preferably also one or more components selected from vitamins, minerals, trace elements and fibers (preferably soluble fibers), wherein component (a) is present in an amount of 0.5 to 10g per 100g of formula diet.

**Patentansprüche**

1. Verwendung mindestens einer Aminosäure, ausgewählt aus der Gruppe, bestehend aus Glycin, Alanin und Serin, oder der physiologisch verträglichen Salze davon, zur Herstellung eines Medikaments oder einer Nährstofformulierung zur enteralen Verabreichung zur Verminderung der Gehalte an Tumornekrosefaktor (TNF) bei Patienten, bei denen die Spiegel über diejenigen erhöht sind, die physiologische Homöostase und lokale Entzündung steuern.

2. Verwendung nach Anspruch 1, wobei die Verminderung der TNF-Gehalte durch Hemmung oder Verminderung von:

    (i) Tumornekrosefaktor-(TNF)-Produktion durch Zellen vom Makrophagentyp;

(ii) Freisetzung von TNF aus Zellen vom Makrophagentyp; und/oder

(iii) Bindung von TNF durch TNF-Rezeptoren erzielt wird.

3. Verwendung nach Anspruch 1 oder 2 zur Verhütung oder Verringerung des Risikos einer Lebererkrankung infolge von exzessivem Alkoholkonsum und intestinaler und pankreatischer Störungen als Ergebnis davon.

4. Verwendung nach Anspruch 1 oder 2 zur Verhinderung oder Verminderung von TNF-induzierter Fremdtransplantatabstoßung.

5. Verwendung nach Anspruch 1 oder 2 zur Behandlung einer Erkrankung oder eines Zustands, ausgewählt aus der folgenden Gruppe, bestehend aus: Osteoporose, Arthritis, Metastasen, Lungenerkrankungen einschließlich adultes Atemnotsyndrom (ARDS), entzündliche Darmerkrankungen einschließlich ulzerative Kolitis und Morbus Crohn, Koronararterienerkrankung, alkohol- oder vireninduzierte Leberzirrhose, Sepsis einschließlich Endotoxinschock, durch Hypoxie bedingte Reperfusionsschädigung, Auszehrungssyndrom, beobachtet bei AIDS- und Krebspatienten, Hepatitis, Nephritis, pathologische Angiogenese, Postbehandlungssyndrome nach Bestrahlungen oder Chemotherapiebehandlungen einschließlich erfolgter Knochenmarkschädigung, Organabstoßung, Trauma, postischämische Herzschädigung, Geschwüre, degenerative Zustände, wie Motorneuronenerkrankung und multiple Sklerose, die mit dem Nervensystem in Zusammenhang stehen, Gehirnschädigung und Entzündung, Pankreatitis, Nierenversagen, Diabetes, Schlaganfall, Asthma, Infektion, chronische Lebererkrankungen und Arteriosklerose.

6. Verwendung nach Anspruch 5 zur Minimierung der Wirkungen von Endotoxämie, durch Hypoxie induzierter Reperfusionsschädigung und Infektion.

7. Verwendung nach Anspruch 6 zur Verringerung oder Verhütung des Mortalitätsrisikos durch Verringerung der Risiken von Endotoxinschock und Sepsis.

8. Medikament oder Nährstofformulierung, umfassend wirksame Mengen von:

(a) einer oder mehreren Aminosäure(n), ausgewählt aus der Gruppe, bestehend aus Glycin, Alanin und Serin in freier Form oder in Form eines physiologisch verträglichen Salzes, oder Gemischen davon (Komponente (a)) in Kombination mit einer oder mehreren Komponente(n), ausgewählt aus

(b) $\omega$-3-PUFAs, sofern erwünscht, im Gemisch mit $\omega$-6-PUFAs (Komponente (b));

(c) L-Arginin oder anderen physiologisch verträglichen Komponenten, die mit der Synthese von Polyaminen im Zusammenhang stehen, oder Gemischen davon (Komponente (c));

(d) einer Quelle für eine Nucleobase (Komponente (d)), wobei die Komponente (a) und ggf. die Komponente (c) die einzigen Aminosäuren in dem Medikament oder der Nährstofffformulierung sind, die als freie Aminosäuren oder in Form eines physiologisch verträglichen Salzes der Aminosäure zugesetzt werden.

9. Medikament oder Nährstofformulierung nach Anspruch 8, umfassend in einer Dosiseinheit

(a) 1,5 bis 80 Gewichtsteile einer oder mehrerer Aminosäure(n), ausgewählt aus der Gruppe, bestehend aus Glycin, Alanin und Serin, in freier Form oder in Form eines physiologisch verträglichen Salzes, oder Gemische davon in Kombination mit einer oder mehreren Verbindungen, ausgewählt aus

(b) 2 bis 5 Gewichtsteilen polyungesättigte $\omega$-3-Fettsäuren;

(c) 7,5 bis 20 Gewichtsteilen L-Arginin oder L-Ornithin oder Gemische davon; und

(d) 1,7 bis 2,0 Gewichtsteile RNA.

10. Medikament oder Nährstofformulierung nach Anspruch 8 oder 9, umfassend die Komponente (a) in Kombination mit der Komponente (c).

11. Medikament oder Nährstofformulierung nach Anspruch 8 oder 9, umfassend die Komponente (a) in Kombination mit den Komponenten (b) und (c).

12. Medikament oder Nährstofformulierung nach Anspruch 8 oder 9, umfassend die Komponente (a) in Kombination mit den Komponenten (b), (c) und (d).

13. Medikament oder Nährstofformulierung nach Anspruch 8, die eine Formuladiät ist, die weiterhin eine Quelle für Kohlenhydrate, für Lipidfett (Fettquelle) und Protein (Stickstoffquelle) und bevorzugt auch eine oder mehrere Komponenten, ausgewählt aus Vitaminen, Mineralien, Spurenelementen und Fasern (bevorzugt lösliche Fasern) umfaßt, wobei die Komponente (a) in einer Menge von 0,5 bis 10 g pro 100 g Formuladiät vorhanden ist.

**Revendications**

1. Utilisation d'au moins un aminoacide choisi parmi le groupe comprenant la glycine, l'alanine et la sérine, ou ses sels physiologiquement acceptables, pour la préparation d'un médicament ou d'une formulation nutritionnelle pour l'administration entérale pour la diminution des taux du facteur de nécrose tumorale (TNF) chez des patients où lesdits taux sont élevés au-delà de ceux qui provoquent l'homéostasie physiologique et l'inflammation locale.

2. Utilisation selon la revendication 1, où une telle diminution des taux du TNF est obtenue par inhibition ou diminution de:

   (i) la production du facteur de nécrose tumorale (TNF) par des cellules du type macrophage;
   (ii) la libération du TNF à partir des cellules du type macrophage; et/ou
   (iii) la liaison du TNF par des récepteurs du TNF.

3. Utilisation selon la revendication 1 ou 2, pour empêcher ou réduire le risque de maladie du foie due à une consommation excessive d'alcool et les troubles intestinaux et pancréatiques en résultant.

4. Utilisation selon la revendication 1 ou 2, pour la prévention ou la diminution du rejet de l'allogreffe induit par le TNF.

5. Utilisation selon la revendication 1 ou 2, pour le traitement d'une maladie ou d'une condition choisie parmi le groupe suivant comprenant: l'ostéoporose; l'arthrite; les métastases; les maladies pulmonaires incluant le syndrome de la détresse respiratoire chez l'adulte (SDRA); les maladies intestinales inflammatoires incluant la colite ulcérative et la maladie de Crohn; la maladie des artères coronaires; la cirrhose du foie induite par l'alcool ou un virus; la septicémie y compris le choc endotoxique; l'hypoxie-les lésions consécutives au rétablissement du flux sanguin; le syndrome du dépérissement observé chez les sidéens et les cancéreux; l'hépatite; la néphrite; l'angiogénèse pathologique; les syndromes de post-traitement suite à des traitements par des rayons ou par chimiothérapie, y compris les lésions de la moelle osseuse; le rejet d'organes; le traumatisme; l'accident cardiaque post-isché-mique; les ulcères; les conditions dégénératives telles que la maladie du neurone moteur et la sclérose en plaques qui sont associées au système nerveux; les accidents cérébraux et l'inflammation; la pancréatite; l'insuffisance rénale; le diabète; l'attaque; l'asthme; l'infection; les maladies chroniques du foie et l'artériosclérose.

6. Utilisation selon la revendication 5, pour minimiser les effets de l'endotoxémie, de l'hypoxie-des lésions consécutives au rétablissement du flux sanguin et de l'infection.

7. Utilisation selon la revendication 6, pour réduire ou empêcher le risque de mortalité en réduisant les risques de choc endotoxique et de septicémie.

8. Un médicament ou une formulation nutritionnelle comprenant des quantités efficaces de:

   (a) un ou plusieurs aminoacides choisis parmi le groupe comprenant la glycine, l'alanine et la sérine, sous forme libre ou d'un sel physiologiquement acceptable, ou leurs mélanges (composant (a)) en association avec un ou plusieurs composants choisis parmi
   (b) les PUFA oméga-3, si désiré, en mélange avec des PUFA oméga-6 (composant (b));
   (c) la L-arginine ou d'autres composés physiologiquement acceptables associés à la synthèse des polyamines, ou leurs mélanges (composant (c));
   (d) une source de nucléobase (composant (d));

   où le composant (a) et éventuellement le composant (c) sont les seuls aminoacides dans le médicament ou la formulation nutritionnelle qui sont ajoutés comme aminoacides libres ou sous forme d'un sel physiologiquement acceptable dudit aminoacide.

9. Le médicament ou la formulation nutritionnelle selon la revendication 8, comprenant, par dose unitaire

   (a) 1,5 à 80 parties en poids d'un ou de plusieurs aminoacides choisis parmi le groupe comprenant la glycine, l'alanine et la sérine, sous forme libre ou sous forme d'un sel physiologiquement acceptable, ou de leurs mélanges,

en combinaison avec un ou plusieurs composés choisis parmi

(b) 2 à 5 parties en poids d'acides gras polyinsaturés oméga-3;

(c) 7,5 à 20 parties en poids de L-arginine ou de L-ornithine, ou de leurs mélanges; et

(d) 1,7 à 2,0 parties en poids d'ARN.

10. Le médicament ou la formulation nutritionnelle selon la revendication 8 ou 9, comprenant le composant (a) en association avec le composant (c).

11. Le médicament ou la formulation nutritionnelle selon la revendication 8 ou la revendication 9, comprenant le composant (a) en association avec les composants (b) et (c).

12. Le médicament ou la formulation nutritionnelle selon la revendication 8 ou la revendication 9, comprenant le composant (a) en association avec les composants (b), (c) et (d).

13. Le médicament ou la formulation nutritionnelle selon la revendication 8, qui est une préparation comprenant en outre une source de glycides, de lipides (source de graisse) et de protéine (source azotée) et de préférence également un ou plusieurs composants choisis parmi les vitamines, les éléments minéraux, les oligo-éléments et les fibres (de préférence les fibres solubles), où le composant (a) est présent en une quantité de 0,5 à 10 g pour 100 g de préparation.